# EUROPÄISCHE PATENTANMELDUNG

(11) **EP 0 611 547 A1**
(43) Veröffentlichungstag der Anmeldung: **24.08.1994**
(21) Anmeldenummer: 94101092.8
(22) Anmeldetag: 26.01.1994
(51) Int. Cl.: A61B 3/13, A61B 3/00, A61B 19/00, G02B 21/06, G02B 21/12

(54) **Operationsmikroskop**

(30) Priorität: 03.02.1993 DE 9301448 U; 16.09.1993 DE 9314020 U
(71) Anmelder: J.D. Möller Optische Werke GmbH, D-22880 Wedel (DE)
(72) Erfinder: Twisselmann, Lorenz, Dr. Ing., D-25497 Prisdorf (DE)
(74) Vertreter: Richter, Werdermann & Gerbaulet

(57) **Zusammenfassung**

Das Operationsmikroskop (110) mit einer Beleuchtungseinrichtung (113) und einem durch das Hauptobjektiv (111) des Operationsmikroskopes (110) geführten Beleuchtungsstrahlenbündel (118,122) ist mit einer in dem Beleuchtungsstrahlenbündel zwischen der Beleuchtungsquelle und dem zu beleuchtenden Objekt angeordneten Einrichtung zum Schutz des Patientenauges im Bereich der Netzhaut vor schädlicher Lichteinwirkung durch die Beleuchtungsstrahlung während der Augenoperation versehen, wobei diese optische Einrichtung aus einem lichtdurchlässigen Träger (16) mit einem kreisförmigen Filter (17) mit einer Transmission von maximal 60% im Bereich des optisch sichtbaren Lichtes von 400-760 nm und einer Transmission von maximal 10% im UV-Bereich unter 400 nm, wobei sich der Filter (17) im eingeschwenkten Zustand in einer zur Objektebene (20) konjugierten Ebene befindet und der kreisförmige Bereich kongruent auf die Patientenpupille abgebildet wird, oder aus einem Filter besteht, der für mindestens jeweils eine Wellenlänge unter 400 nm und eine weitere über 400 nm im sichtbaren Spektralbereich eine geringere Transmission aufweist.

## Beschreibung

Die Erfindung betrifft ein Operationsmikroskop mit einer Beleuchtungseinrichtung mit einem durch das Hauptobjektiv des Operationsmikroskopes geführten Beleuchtungsstrahlenbündel, in dem eine optische Einrichtung zum Schutz des Patientenauges im Bereich der Netzhaut vor schädlicher Lichteinwirkung durch die Beleuchtungsstrahlung während der Augenoperation zwischen der Beleuchtungsquelle und dem zu beleuchtenden Objekt angeordnet ist.

Operationsmikroskope mit einer Beleuchtungseinrichtung sind bekannt.

In der DE-A-33 39 172 wird das Problem angesprochen, daß bei lichtstarken Augenuntersuchungsgeräten die Gefahr einer zu starken Belastung des Patientenauges im Bereich der Netzhaut bestehen könne, was es erforderlich mache, das Patientenauge vor einer Beleuchtungsbestrahlung zu schützen, die ein unschädliches Maß übersteigt. Hierzu wird zum Schutz vor schädlicher Lichteinwirkung während der Augenoperation unter Aufrechterhaltung einer hinreichend großen Helligkeit auf dem Operationsfeld vorgeschlagen, eine ringförmige Blende in das Beleuchtungsstrahlenbündel ein- und ausschwenkbar anzuordnen, die so dimensioniert ist, daß ihr mittlerer, lichtundurchlässiger Bereich in der Objektebene eine Abschattung erzeugt, die konzentrisch zur Patienten-pupille ist und deren Durchmesser dem Pupillendurchmesser des Patienten entspricht und deren ringförmiger, lichtdurchlässiger Teil die Hornhaut des Patientenauges beleuchtet. Diese Leuchtfeldblende ist hiernach als ringförmige Blende ausgeführt, in deren zentralem Teil sich eine lichtabsorbierende Schicht, d.h. ein Schwarzpunkt, befindet, wobei der Durchmesser des zentralen Teiles der ringförmigen Blende so bemessen ist, daß auf der Hornhaut des Patientenauges eine zentrale Abschattung entsteht, deren Durchmesser dem Pupillendurchmesser des Patienten entspricht. Diese Blende hat allerdings den Nachteil, daß der Pupillenbereich der Hornhaut für den operierenden Arzt nicht mehr sichtbar ist, z.B. wenn er bei einer Katarakt-Operation nach Implantation einer Kunstlinse die notwendige Öffnung mit einer Naht wieder verschließt. Gerade bei dieser Operation ist es besonders wichtig, die Naht so auszuführen, daß durch Verzerrungen der Hornhaut kein Astigmatismus entsteht. Eine völlig abgedunkelte Hornhaut durch die genannte Blende ist jedoch der Beurteilung ihrer Oberflächenform durch den operierenden Arzt entzogen.

Nach der CH-A-665 111 wird in einer entsprechenden Anordnung vorgeschlagen, eine lichtabsorbierende Schicht, beispielsweise einen Schwarzpunkt, vorzusehen. Der Nachteil dieser Lichtabsorptionsschichten besteht darin, daß die auftretende Strahlungsenergie in Wärme umgewandelt wird, so daß die Blende bzw. das Operationsmikroskop je nach eingegebener Strahlungsleistung eine beträchtliche Erwärmung erfährt.

Es ist daher Aufgabe der vorliegenden Erfindung, das eingangs genannte Operationsmikroskop dahingehend zu verbessern, daß einerseits die Beleuchtungsstärke im Pupillenbereich der Hornhaut soweit verringert wird, daß eine Schädigung der Retina in einer angemessenen Operationszeit verhindert wird, andererseits der operierende Arzt im gesamten Pupillenbereich eine gute Ausleuchtung des Operationsfeldes ohne aufwendige Nachstellarbeiten am Mikroskop erhält. Die hierzu notwendige Einrichtung soll leicht handhabbar und konstruktiv möglichst einfach aufgebaut sein, vorzugsweise weiter in der Art, daß auch eine Nachrüstung bereits bestehender Operationsmikroskope möglich ist. Nach einem weiteren Teilaspekt der Aufgabe soll eine weitere Wärmeentwicklung im Operationsmikroskop möglichst vermieden werden. Außerdem soll bei lichtstarken Augenuntersuchungsgeräten die insbesondere durch UV-Anteile auftretende Gefährdung des Patienten - wie auch des Arztauges - vermieden werden, ohne daß der Farbeindruck des natürlichen Lichtes beeinträchtigt wird.

Eine erste Lösung der Aufgabe besteht bei einem Operationsmikroskop in der im Anspruch 1 angegebenen Ausgestaltung.

Danach ist das Operationsmikroskop in der Weise ausgebildet, daß mindestens ein in das Beleuchtungsstrahlenbündel lichtdurchlässiger Träger mit einem kreisförmigen Filter einer auf die Beleuchtungsstärke abgestimmten Transmission von maximal 60% im Bereich des optisch sichtbaren Lichtes von 400 bis 760 nm ein- und ausschwenkbar ist, wobei sich der Filter im eingeschwenkten Zustand in einer zur Objektebene konjugierten Ebene befindet und der kreisförmige Bereich überdeckend auf die geweitete Patientenpupille abgebildet wird und daß der Filter aus einer mattierten Scheibe und/oder mit einer sphärisch divergierend brechenden Fläche ausgestatteten Scheibe besteht. Vorteilhafterweise wirkt der Filter des genannten Trägers wie eine "schützende Sonnenbrille", die jedoch das einfallende Licht nur soweit abdunkelt, daß eine Schädigung der Retina verhindert, andererseits jedoch noch ein hinreichend gut ausgeleuchtetes Operationsfeld des gesamten Auges aufrechterhalten bleibt. Von besonderem Vorteil zum Schutz der Retina ist es, wenn die Transmission im UV-Bereich unter 400 nm besonders gering ist (z.B. 3% bei unter 400 nm , 11% bei unter 500 nm, 20% bei unter 600 nm). Soweit der Filter aus einer mattierten Scheibe und/oder mit einer sphärisch divergierend brechenden Fläche ausgestatteten Scheibe besteht, wird weiterhin eine Wärmeentwicklung verhindert, wie sie bei einer Strahlungsabsorption zwangsläufig entsteht. Stattdessen wird das einfallende Licht im Bereich des Filters zerstreut, was ebenfalls eine hinreichende Schwächung der hindurchgehenden Intensität bewirkt, obwohl die Strahlung als solche erhalten bleibt.

Eine weitere Lösung der Aufgabe besteht bei einem Operationsmikroskop in der im Anspruch 2 angegebenen Ausgestaltung.

Hiernach besteht die erfindungsgemäße Ausgestaltung darin, daß der oder die Filter für mindestens jeweils eine Wellenlänge unter 400 nm und ein weiterer über 400 nm im sichtbaren Spektralbereich eine geringere Transmission aufweist/aufweisen. Bevorzugt wird der bzw. werden die Filter derart ausgewählt, daß die Farbe des durchgelassenen Lichtes unverändert gegenüber der Farbe des eingestrahlten Lichtes ist. Die Filter müssen demnach derart kombiniert werden, daß sie vom optischen Farbeneindruck her komplementär sind, was durch geeignete Wahl der Komplementärfarben und der Farbtiefe, bezogen auf das Beleuchtungslicht, erreicht wird. Soweit durch die verwendeten Filter das Beleuchtungslicht abgeschwächt wird, kann es ohne die Gefahr einer Schädigung der Augen des Operateurs oder des Patienten durch eine höhere Beleuchtungsleistung ausgeglichen werden.

Weitere Ausgestaltungen der Erfindung sind in den Unteransprüchen beschrieben.

So sind vorzugsweise mehrere Träger mit jeweils unterschielichen Filtern separat schwenkbar angeordnet, was die Möglichkeit verschafft, die Filterkombination oder den Filter auszuwählen, der hinsichtlich der Ausleuchtung und der Geometrie adäquat ist. Vorzugsweise besitzen die unterschiedlichen Filter unterschiedliche Kreisdurchmesser, so daß auch unterschiedlichen Pupillenaufweitungen Rechnung getragen wird. Die Kreisdurchmesser betragen in der Abbildung zwischen 6 und 12 mm, vorzugsweise liegen sie bei 8 mm.

Für die Filter selbst gibt es verschiedene Ausführungsformen: In einer ersten Ausführungsform ist die Transmission im kreisförmigen Filter an jeder Stelle gleich groß, so daß ein "homogen gefilterter" Bereich auf die Hornhaut des Patienten geschaffen wird. Alternativ hierzu ist es jedoch auch nach einer weiteren Ausgestaltung der Erfindung möglich, Filter zu verwenden, die eine im Zentrum geringste Transmission, beispielsweise maximal 20%, und eine radial nach außen zunehmende Transmission, beispielsweise maximal 60%, aufweisen. Die Transmission kann abgestuft radial von innen nach außen, also z.B. zweistufig geändert werden, wobei die Pupille stark abgedunktelt wird und das sich hierum erstreckende Beleuchtungsfeld schwach, z.B. mit 10% gefiltert wird. Alternativ hierzu kann auch der Filtergrad radial von innen nach außen kontinuierlich abnehmen. Kombinationen dieser kontinuierlichen Filterung mit einer Abstufung sind ebenfalls möglich.

Nach einer weiteren Ausführungsform der Erfindung sind der oder die Filter Absorptions- oder Dichroitische-(Interferenz-) Filter. Mit Hilfe solcher Filter ist es möglich, UV-Anteile und entsprechende Farben zu kompensieren, so daß insgesamt die Farbe des Lichtes vor Filterung gleich der Farbe des Lichtes nach der Filterung erscheint. Prinzipiell ist es sowohl möglich, UV-Anteile sowie die komplimentären Anteile im sichtbaren Spektrum durch einen einzigen Filter oder durch mehrere hintereinander geschaltete Filter zu entfernen. Vorzugsweise bestehen die Filter aus Folien oder Schichten, die mit einem Träger verbunden sind.

Ausführungsbeispiele der Erfindung sind in den Zeichnungen dargestellt. Es zeigen
Fig. 1 einen Querschnitt durch das Beleuchtungsteil eines Operationsmikroskopes mit eingezeichnetem Strahlengang in Verbindung mit einem beleuchteten Objekt in einer ersten Ausgestaltung,
Fig. 2 eine Seitenansicht eines kreisförmigen Filters mit mattierter Oberfläche,
Fig. 3 eine Seitenansicht eines Filters mit divergierend brechender Fläche,
Fig. 4 einen Querschnitt durch den Beleuchtungsteil eines Operationsmikroskopes mit eingezeichnetem Strahlengang in Verbindung mit einem beleuchteten Objekt, in einer zweiten Ausgestaltung, und
Fig. 5a,5b und 5c verschiedene Ausführungsformen der Filter in Seitenansichten.

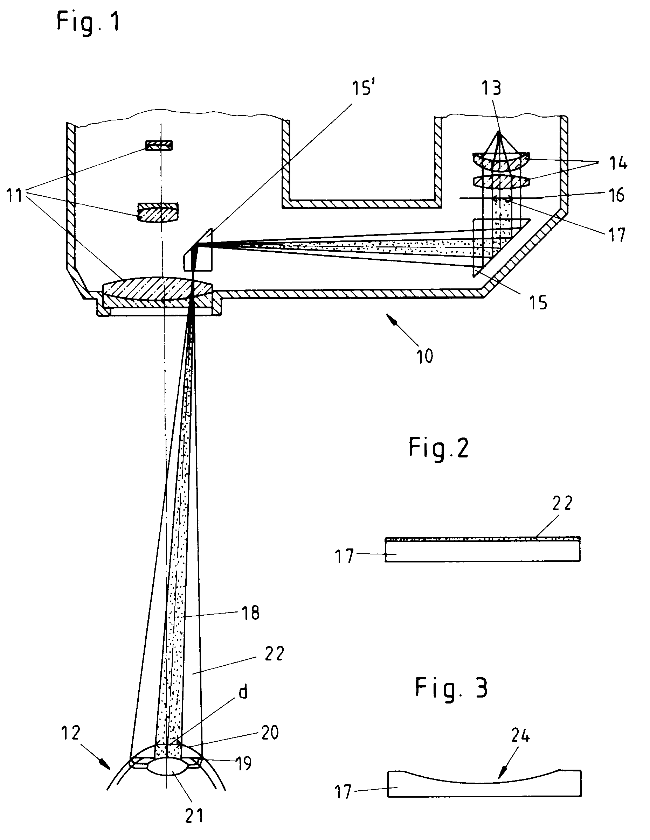

Das in Fig.1 dargestellte Operationsmikroskop für Augenoperationen 10 weist eine aus mehreren Linsen bestehende Linsenanordnung 11 sowie zur Beleuchtung des Untersuchungsobjektes - hier des Auges 12 - eine Beleuchtungsquelle 13 auf, deren Licht nach paralleler Ausrichtung durch das Linsensystem 14 über Umlenkprismen 15 und 15' so gerichtet wird, daß es das Auge 12 voll ausleuchtet. In diesem Beleuchtungsstrahlengang ist zwischen der Linsenanordnung 14 und dem ersten Umlenkprisma 15 ein lichtdurchlässiger Träger 16 ein- und ausschwenkbar angeordnet. Dieser Träger 16 besitzt einen zentralen kreisförmigen Bereich 17 als Filter, der eine Transmission von 20% im Bereich des optisch sichtbaren Lichtes von 400-760 nm hat. Der Träger 16 ist im eingeschwenkten Zustand derart angeordnet, daß das hinter dem Filter 17 verlaufende Strahlenbündel 18 einen Durchmesser d im Bereich der Hornhaut 20 hat, daß die gesamte, durch die Iris 19 begrenzte Pupille (siehe Augenlinse 21) kongruent abgeschattet wird. Das darumliegende Beleuchtungsfeld des Strahlenbündels 22 gelangt hingegen ungefiltert auf die Hornhaut 20 des Auges 12.

Wie aus Fig. 2 ersichtlich, besitzt der Filter 17 eine mattierte Oberfläche 23, an der das Licht gestreut wird. Alternativ und wie Fig.3 zu entnehmen, kann der Filter 17 auch eine sphärische Fläche 24 besitzen, die sich entweder über den gesamten Filterkreis oder einen Teil hiervon erstreckt. Die sphärische Fläche 24 wirkt wie eine Zerstreuungslinse, d.h. sie bricht das einfallende Licht zu einem divergierenden Strahlenbündel. Der Grad der Divergenz bestimmt gleichzeitig die Transmission.

Es sind auch Kombinationen einer mattierten Oberfläche und einer sphärischen Fläche 24 im Sinne der vorliegenden Erfindung ebenso möglich wie beidseitig mattierte Filter 17 und/oder beidseitig angeordnete sphärische Flächen.

Es können auch mehrere lichtdurchlässige Träger 16 mit jeweiligen Filtern 17 übereinander angeordnet sein, die entweder einzeln oder gruppenweise in den Strahlengang anstelle des einzelnen dargestellten Trägers 16 eingeschwenkt werden können. Diese Träger 16 können auch um ihren jeweiligen Mittelpunkt mehrere Filterstufen unterschiedlicher Transmissionsgrade aufweisen oder in dem den Kreis 17 umrandenden Bereich schwache Absorptionen des Lichtes, auch partiell ermöglichen. Jeder der beschriebenen Filter einzeln oder Kombinationen mit einem zweiten ermöglicht jedenfalls eine individuelle Einstellung des begrenzt abschattierten Pupillenbereiches und eine jeweils angemessene Ausleuchtung.

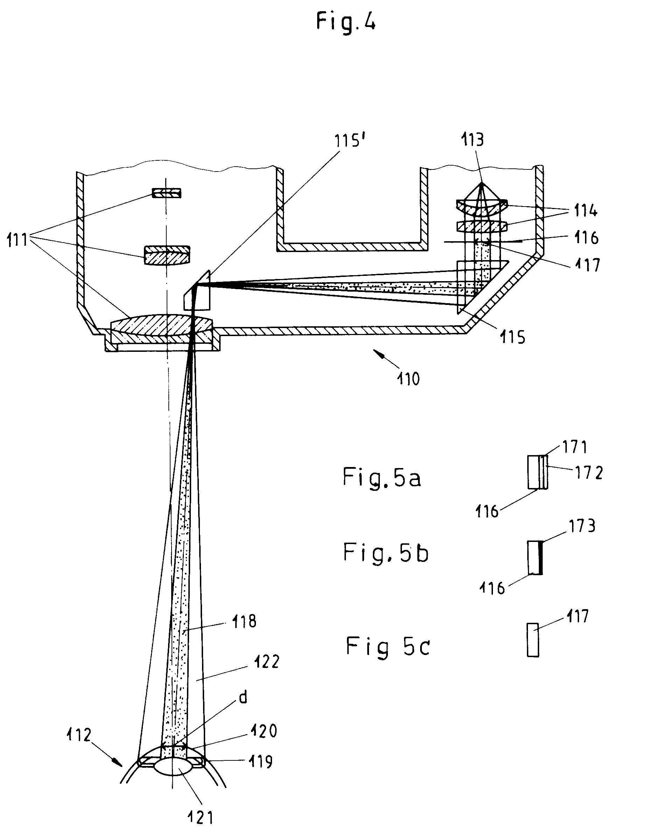

Auch das in Fig.4 dargestellte Mikroskop 110 weist eine aus mehreren Linsen bestehende Linsenanordnung 111 sowie zur Beleuchtung des Untersuchungsobjektes - hier des Auges 112 - eine Beleuchtungsquelle 113 auf, deren Licht nach paralleler Ausrichtung durch das Linsensystem 114 über Umlenkprismen 115 und 115' so gerichtet wird, daß es das Auge 112 voll ausleuchtet. In diesen Beleuchtungsstrahlengang ist zwischen der Linsenanordnung 114 und dem ersten Umlenkprisma 115 ein lichtdurchlässiger Träger 116 ein- und ausschwenkbar angeordnet. Dieser Träger 116 besitzt einen zentralen kreisförmigen Bereich 117 als Filter, der im UV-Bereich sowie für mindestens eine Wellenlänge im optischen Bereich ( 400 nm bis 760 nm) eine geringere Transmission aufweist als für die übrigen Wellenlängenbereiche, wobei die Transmission unterhalb 400 nm in der hier vorliegenden Anordnung weniger als 10% der durchschnittlichen Transmission im Sichtbaren beträgt. Mit UV-Bereich sind bevorzugterweise durchweg die Wellenlängen im Bereich unterhalb 400 nm zu verstehen. Im sichtbaren Bereich wird zur Farbkorrektur ein Teilbereich zwischen 560 nm und 600 nm herausgefiltert. In diesem Teilbereich sinkt die Transmission auf 20-30% der durchschnittlichen Transmission.

Es können auch mehrere lichtdurchlässige Träger mit jeweiligen Filtern 117 übereinander angeordnet sein, die entweder einzelnen oder gruppenweise in den Strahlengang anstelle des einzelnen dargestellten Trägers 116 eingeschwenkt oder ständig im Strahlengang installiert werden können. Der Filter 117 bzw. der Träger 116 mit Filter 117 kann auch an anderer Stelle zwischen der Beleuchtungsquelle 113 und dem Lichtaustritt aus dem Linsensystem 111 angeordnet werden, beispielsweise als Vorsatzlinse des Operationsmikroskopes 110.

Es können auf einem Absorptionsglas 116 auch einzelne Farbfolien 171,172, z.B. der Farben Margenta und Cyan aufgeklebt sein (Fig.5a). In Fig. 5b sind entsprechende dichtroitische Schichten 173 auf einem Absorptionsglas 116 aufgedampft. Schließlich können entsprechend 5c auch homogene Glas- oder Kunststoffplatten 117 mit eingelagerten Farbstoffen für die UV-Absorption und die Margenta/Cyan Farbabsorption verwendet werden.

## Patentansprüche

1. Operationsmikroskop (10) mit einer Beleuchtungseinrichtung (13) mit einem durch das Hauptobjektiv (11) des Operationsmikroskopes (10) geführten Beleuchtungsstrahlenbündel (18,22), in dem eine optische Einrichtung zum Schutz des Patientenauges im Bereich der Netzhaut vor schädlicher Lichteinwirkung durch die Beleuchtungsstrahlung während der Augenoperation zwischen der Beleuchtungsquelle und dem zu beleuchtenden Objekt angeordnet ist, dadurch gekennzeichnet, daß als optische Einrichtung mindestens ein in das Beleuchtungsstrahlenbündel (18,22) lichtdurchlässiger Träger (16) mit einem kreisförmigen Filter (17) mit einer Transmission von maximal 60% im Bereich des optisch sichtbaren Lichtes von 400-760 nm, und einer Transmission von maximal 10% im UV-Bereich unter 400 nm, ein- und ausschwenkbar ist, wobei sich der Filter (17) im eingeschwenkten Zustand in einer zur Objektebene (20) konjugierten Ebene befindet und der kreisförmige Bereich kongruent auf die Patientenpupille abgebildet wird, und daß der Filter (17) aus einer mattierten Scheibe und/oder mit einer sphärisch divergierend brechenden Fläche ausgestatteten Scheibe besteht.

2. Operationsmikroskop (110) mit einer Beleuchtungseinrichtung (113) und einem durch das Hauptobjektiv (111) des Operationsmikroskopes (110) geführten Beleuchtungsstrahlenbündel (118,122), in dem eine optische Einrichtung zum Schutz des Patientenauges im Bereich der Netzhaut vor schädlicher Lichteinwirkung durch die Beleuchtungsstrahlung während der Augenoperation zwischen der Beleuchtungsquelle und dem zu beleuchtenden Objekt angeordnet ist, dadurch gekennzeichnet, daß die optische Einrichtung aus mindestens einem Filter (117) besteht, wobei der oder die Filter (117) für mindestens jeweils eine Wellenlänge unter 400 nm und eine weitere über 400 nm im sichtbaren Spektralbereich eine geringere Transmission aufweist/aufweisen.

3. Operationsmikroskop nach Anspruch 1, dadurch gekennzeichnet, daß mehrere Träger (16) mit jeweils unterschiedlichem Filter (17) vorgesehen und jeder separat schwenkbar angeordnet ist.

4. Operationsmikroskop nach Anspruch 3, dadurch gekennzeichnet, daß die unterschiedlichen Filter (17) unterschiedliche Kreisdurchmesser aufweisen.

5. Operationsmikroskop nach Anspruch 4, gekennzeichnet durch einen Kreisdurchmesser ihrer Abbildungen zwischen 6 mm - 12 mm, vorzugsweise 8 mm.

6. Operationsmikroskop nach einem der Ansprüche 1,3 bis 5, dadurch gekennzeichnet, daß die Transmission im kreisförmigen Filter (17) an jeder Stelle gleich groß ist.

7. Operationsmikroskop nach einem der Ansprüche 1, 3 bis 6, dadurch gekennzeichnet, daß die Filter (17) eine im Zentrum geringste Transmission, vorzugsweise von maximal 20% und einer radial nach außen zunehmenden Transmission, vorzugsweise bis maximal 60% ,aufweisen.

8. Operationsmiskroskop nach Anspruch 2, dadurch gekennzeichnet, daß die Filterung die Farbe des durchgelassenen Lichtes unverändert läßt.

9. Operationsmikroskop nach Anspruch 2 oder 8, dadurch gekennzeichnet, daß der oder die Filter Absoprtions- oder Dichroitische- (Interferenz) Filter sind.

10. Operationsmikroskop nach einem der Ansprüche 1,8 und 9, dadurch gekennzeichnet, daß die Filter aus Folien oder Schichten bestehen, die mit einem Träger (116) verbunden sind.
